# EUROPEAN PATENT APPLICATION

(11) **EP 0 743 051 A2**
(43) Date of publication of application: **20.11.1996**
(21) Application number: 96302942.6
(22) Date of filing: 26.04.1996
(51) Int. Cl.: A61F 9/08

(54) **Apparatus and method for providing an image output to optic nerves**

(30) Priority: 26.04.1995 JP 127203/95
(71) Applicant: Yamamoto, Hiroshi, Kitauwe-gun, Ehime Prefecture (JP); TECNO ISHII CO., LTD., Matsuyama-City, Ehime Prefecture (JP)
(72) Inventor: Ishii, Touru, Matsuyama-City, Ehime-Prefecture (JP); Yamamoto, Hiroshi, Kitauwa-gun, Ehime Prefecture (JP)
(74) Representative: Warren, Anthony Robert

(57) **Abstract**

The invention relates to an apparatus and method for providing output video signals representative of a visual image, and applying them to the head of a person, in regions associated with the optic nerves, to enable the person to mentally visualise or recognize the image.

Image signals output by an image output device (10), such as a video camera, are modulated with low-frequency square waves output by square wave output and modulation circuits (12, 19). The modulated output is applied by electrodes (15) to portions of the temples, acted upon by the optic nerve, of a person's head.

If low-frequency square wave signals are applied to the temple portions, they are sensed by the optic nerve, and the person can mentally visualise or recognize a square screen of light, and scanning lines on that screen. For that reason, if video signals are applied after modification, e.g. modulation, with such low-frequency square wave signals, an image is directly sensed by the optic nerve, making it possible, not only for healthy persons, but also blind or partially sighted persons, to recognize the image, thus also making is possible to use the human body as a display.

## Description

This invention relates to an image output device, system or apparatus which is capable of visualizing an image (e.g. a subject or figure) and using a human body as a display by directly applying video signals to the human body, and also relates to an associated method.

Conventionally, CRTs, liquid crystals, electric discharge tubes, etc. are used as devices for outputting and displaying images, and, with all such display devices, recognition of an image (e.g. figures) is possible only with the existence of the visual power of the viewer's eyes. However, persons without sight, or with impaired sight or visual power (e.g. with disorders of, or associated with, the lens system or retina system), cannot recognize the images displayed by such display devices. Namely, it is difficult if not impossible for visually handicapped or blind persons to recognize such images.

It is an object of this invention to provide an image output device or apparatus, and method, by means of which an image (subject) may be visualised, making it possible not only for healthy persons, but also persons without visual power or with impaired visual power (e.g. with disorders of the lens system or retina system), to recognize the image, using the human body as a display device by directly applying video signals to the human body.

According to one aspect, this invention is characterised by an optic nerve image output apparatus or device provided with image output means for outputting image signals, square wave output means for outputting relatively low-frequency square waves, modulation means for modulating image signals output by said image output means with relatively low-frequency square waves output by said square wave output means, and output means for outputting signals modulated by said modulation means at a portion of a person's head acted upon by an optic nerve.

From another aspect, this invention is characterised by an optic nerve image output method for applying video signals to a portion acted upon by an optic nerve at the temples of a person's head, by modulating the signals with low-frequency square waves.

If relatively low-frequency, generally rectangular or square wave signals are applied externally to the portion of a person's head acted upon by, adjacent to, or associated with, the optic nerve, the optic nerve can be sensed or stimulated by the signals, to cause the person to experience a mental picture of a generally rectangular or square screen of or by light, and scanning lines on that screen. For that reason, if video signals are applied after modification (e.g. by modulation, or including modulation) with such low-frequency square wave signals, the scanning lines visualized on the light screen in the person's perceived mental picture form an image, making is possible for the person, in effect, to visually recognize that image.

Moreover, in the case where square waves are formed as a continuous alternating wave form in which the polarity is alternatively switched, an effective light or an image of light can be obtained visually or recognised or sensed. Furthermore, when square waves are formed at a low frequency of 15 to 25 Hz, an effective luminance (brightness) can be obtained or sensed. The image becomes white with excessive luminance, and rough, at 15 Hz or below, but becomes dark with low luminance at 25 Hz or above.

As explained above, the apparatus and method embodying this invention makes it possible, not only for healthy persons, but also for persons with poor visual power, or blind persons, in effect, to visually recognize images, i.e. mentally sense or visualise images, because the images are sensed or felt directly by the optic nerve or nerves. Therefore the invention enables the human body to be used as a display.

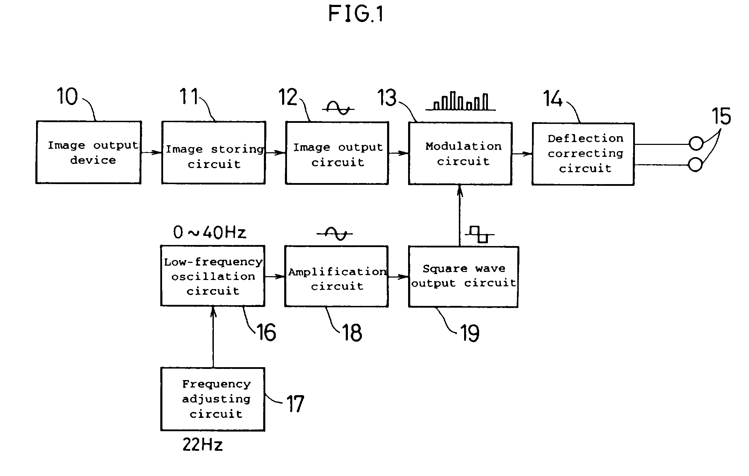

One embodiment of this invention will now be described with reference to the accompanying drawings, in which Fig. 1, the single Figure, is a block diagram of the optic nerve image output apparatus.

The optic nerve image output apparatus or system shown in Fig. 1 includes an image output device 10 comprising or including equipment capable of outputting video signals, such as a video camera, laser disc player, personal computer (P.C.) or "Family Computer", etc., which outputs video signals. An image storage circuit 11 stores the video signals in each visually caught single image, and the video signals of this single image are converted into a single static image.

An image output circuit 12 extracts the video signals for a single static image (which may correspond to one or more fields, frames or equivalent) from the image storage circuit 11 in the preceding stage and outputs them for a prescribed time, for example 2 seconds, and repeats this video signal extraction and output cycle. Because storing of images becomes insufficient with a static image of less than 2 seconds, a static image of 2 to 8 seconds is desirable to obtain a natural storing state.

A modulation circuit 13 modulates the above-mentioned video signals, with square waves to be described later, into signals which can, in effect, be visually sensed or caught by an optic nerve. The modulation circuit 13 is connected to a deflection correcting circuit 14 which corrects the parallelism of scanning lines produced as a vision in the optic nerve and, in particular, corrects the scanning lines appearing lower on the right to be parallel. The deflection correcting circuit 14 is connected to electrodes 15 which comprise output means for applying modified and corrected video signals to the portions acted upon by the optic nerves at both temples of the human head.

A low-frequency oscillation circuit 16 provides low-frequency signals over a range of 0 to 40 Hz, and a frequency adjusting circuit 17 controls the frequency of the low-frequency output signals of the low-frequency oscillation circuit 16, for example by adjusting the frequency to 22 Hz. An amplification circuit 18 amplifies the low-frequency (e.g. 22 Hz) signals, while a square wave output circuit 19 outputs, to the modulation circuit 13, continuous alternating square waves of which the polarity is alternately switched.

To explain the action of an optic nerve image output apparatus constructed in this way, when a relatively low-frequency square wave signal is applied to the portions acted upon by the optic nerves at both temples of the human head, the optic nerve senses, or is stimulated to produce, a mental picture in the form of a stable square screen of light, and scanning lines visualized on that screen. For that reason, if the video signal is applied after modulation with a low-frequency square wave signal, the scanning lines form an image, making it possible to recognize or visualise that image.

The screen of light visually caught by the optic nerve is a square of stable shape, and the number of scanning lines in this screen is stable in correspondence to the frequency of the square waves. For example, at a frequency of 15 Hz, 15 scanning lines appear, and, similarly, 22 scanning lines appear at 22 Hz, 25 scanning lines appear at 25 Hz, and 44 scanning lines appear at 44 Hz. However, the length (range) of the scanning lines remains the same (fixed value) at any frequency. For that reason, the storage capacity for a single static image of the image storing device 11 is set in correspondence to this number and length of the scanning lines.

In particular, as video signals are output from the image output device 10, the image storing device 11 stores the video signals in each visually caught single image, and the image output circuit 12 reads the video signals for a single static image from the image storing device 11 every 2 seconds and outputs them for 2 seconds. The modulation circuit 13 modulates the video signals with square waves, while the deflection correcting circuit 14 corrects the parallelism and outputs the signals to the electrodes 15. This output has, for example, a frequency of 22 Hz, a voltage of 2 V and a current intensity of 100 µA.

The above-mentioned electrodes 15 can apply the said video signals to each portion acted upon by the associated optic nerve at both temples of the human head, by being put in electrical contact with the skin of that portion.

As a result, the optic nerve can sense what the brain recognizes or interprets as a square screen of or by light, can further sense scanning lines on that square screen, and, because these scanning lines are visualized or modified by video signals, can visually recognize or interpret them as an image. That recognized image can be visualized like a motion picture fed frame-by-frame or field-by-field at 2-second intervals. Such visualization of an image makes it possible to use the human body as a display.

As indicated in the above-described embodiment, by forming a square wave output from the square wave output circuit 19 as a continuous alternating waveform in which the polarity is alternately switched, it is possible to visually obtain an effective mental image or picture of light, or recognizable as light. Moreover, if the square waves are formed with low-frequency waves of 15 to 25 Hz, an effective or improved perceived luminance (brightness) can be obtained. The perceived image becomes white with excessive luminance, and rough, at 15 Hz or below, but becomes dark with low luminance at 25 Hz or above. However, if the frequency is set for 44 Hz, visual recognition of the image is possible, although inferior in luminance, and the image becomes sharper because of the increase in the number of scanning lines to 44. For that reason, the latter frequency can be used when priority is given to image sharpness or resolution rather than to luminance.

While the image in the aforementioned embodiment is a motion picture sent frame-by-frame at 2-second intervals, in the visualization of ordinary motion pictures, it is possible to control the video signals output from the image output device 10 to an output for one scanning line and an output for one image by means of the image output circuit 12.

The deflection correcting circuit may be adapted to correct for discrepancies in alignment or parallelism between the scanning lines perceived in the or each perceived light frame, and/or to correct for discrepancies in alignment, parallelism and/or coincidence between the scanning lines perceived in different light frames, for example resulting from the different electrodes and different, i.e. left and right, optic nerves.

## Claims

1. An optic nerve image output apparatus or device provided with image output means (10) for outputting image signals, square wave output means (19) for outputting low-frequency square waves, modulation means (13) for modulating image signals output by said image output means (10) with low-frequency square waves output by said square wave output means (19), and output means (15) for outputting signals modulated with said modulation means at the portion or portions, acted upon by optic nerve, at the head on the human body side.

2. An optic nerve image output method for applying video signals to the portion or portions, acted upon by optic nerve, at the temple or temples on the human body side by modulating them with low-frequency square waves.

3. An apparatus as defined in claim 1, or a method as defined in claim 2, wherein said square waves are formed in to a continuous alternating waveform in which the polarity is alternately switched.

4. An apparatus or method as defined in claim 1, 2 or 3, wherein said square waves are formed as low-frequency waves of 15 to 25 Hz.

5. An apparatus or method as defined in any preceding claim, wherein the video signals for a single static image, prior to modulation, are stored for a predetermined time and then output for a predetermined time on an image-by-image, field-by-field, or frame-by-frame basis, and the storage and output cycle is repeated.

6. An apparatus or method as defined in claim 5, wherein the prescribed storage time, and/or the prescribed output time, is in the range of 2 to 8 seconds.

7. An apparatus or method as defined in any preceding claim, wherein the modulated signals, prior to application to the head portion or portions, are modified to correct for parallelism of scanning lines in the resultant visualised images.
